# EUROPEAN PATENT APPLICATION

(11) **EP 2 189 528 A1**
(43) Date of publication of application: **26.05.2010**
(21) Application number: 08792323.1
(22) Date of filing: 01.08.2008
(51) Int. Cl.: C12N 15/09, C07K 14/435, C07K 19/00, C12N 1/15, C12N 1/19, C12N 5/10

(54) **FLUORESCENT PROTEIN WITH DEEP BLUE COLOR**

(30) Priority: 03.08.2007 JP 2007203300
(71) Applicant: National University Corporation Hokkaido University, Sapporo-shi Hokkaido 060-0808 (JP)
(72) Inventor: The designation of the inventor has not yet been filed
(74) Representative: Cabinet Plasseraud
(86) International application number: PCT/JP2008/064266
(87) International publication number: WO 2009/020197

(57) **Abstract**

The present invention provides an artificial mutant of GFP having a novel emission peak, i.e., a fluorescent protein having an emission peak at 424 nm comprising an amino acid sequence represented by SEQ ID NO: 1, in which each of the amino acid residues at the 66th position and the 175th position is replaced and at least one of the amino acid residues at the 72nd position and the 206th position is further replaced, or a fluorescent protein having an emission peak at 424 nm and a pH-independent fluorescence intensity, in which each of the amino acid residues at the 65th, 145th, 148th, 46th and/or 203rd positions is further substituted. The fluorescent protein of the invention emits fluorescence having an emission peak at 424 nm and can be visually distinguished by its ultramarine color from other fluorescent proteins. The fluorescent protein has a pH-independent fluorescence intensity which is not affected by pH changes.

SEQUENCE LISTING

<110> National University Corporation Hokkaido University

<120> Ultramarine fluorescent protein

<130> G09-0092

<140> PCT/JP2008/064266
<141> 2008-08-01

<150> JP 2007-203300
<151> 2007-08-03

<160> 33

<170> PatentIn version 3.5

<210> 1
<211> 239
<212> PRT
<213> Aequorea victoria

<400> 1

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  2
<211>  720
<212>  DNA
<213>  Aequorea victoria

<400>  2
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgacctg gggcgtgcag tgcttcagcc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacat cagccacaac gtctatatca ccgccgacaa gcagaagaac     480

ggcatcaagg cccacttcaa gatccgccac aacatcgagg acggcagcgt gcagctcgcc     540

gaccactacc agcagaacac ccccatcggc gacggccccg tgctgctgcc cgacaaccac     600

tacctgagca cccagtccgc cctgagcaaa gaccccaacg agaagcgcga tcacatggtc     660

ctgctggagt tcgtgaccgc cgccgggatc actctcggca tggacgagct gtacaagtaa     720

<210> 3
<211> 21
<212> DNA
<213> Artificial sequence

<220>
<223> primer1

<400> 3
cagtgcttca gccgctaccc c 21


<210> 4
<211> 21
<212> DNA
<213> Artificial sequence

<220>
<223> primer2

<400> 4
gaggacggca gcgtgcagct c 21


<210> 5
<211> 21
<212> DNA
<213> Artificial sequence

<220>
<223> primer3

<400> 5
acccagtccg ccctgagcaa a 21


<210> 6
<211> 21
<212> DNA
<213> Artificial sequence

<220>
<223> primer4

<400> 6
accctgacct tcggcgtgca g 21


<210> 7
<211> 21
<212> DNA
<213> Artificial sequence

<220>
<223> primer5

<400> 7

accaccctgc aattcggcgt g                                                21

<210>  8
<211>  21
<212>  DNA
<213>  Artificial sequence

<220>
<223>  primer6

<400>  8
gagtacaacg ggatcagcca c                                                21

<210>  9
<211>  21
<212>  DNA
<213>  Artificial sequence

<220>
<223>  primer7

<400>  9
gggatcagct caaacgtcta t                                                21

<210>  10
<211>  21
<212>  DNA
<213>  Artificial sequence

<220>
<223>  primer8

<400>  10
accctgaagc tcatctgcac c                                                21

<210>  11
<211>  21
<212>  DNA
<213>  Artificial sequence

<220>
<223>  primer9

<400>  11
tacctgagcg tccagtccgc c                                                21

<210>  12
<211>  21
<212>  DNA
<213>  Artificial Sequence

<220>
<223>  primer10

<400>  12
ttcggggtgc tgtgcttcgc c                                                21

<210>  13
<211>  21
<212>  DNA
<213>  Artificial Sequence

<220>
<223>  primer 11

<400>  13
ttcggggtgc tgtgcttcgc c                                                21

<210>  14
<211>  720
<212>  DNA
<213>  Artificial Sequence

<220>
<223>  ECFP-S72A-S175G-A206K

<400>  14
atggtgagca agggcgagga gctgttcacc ggggtggtgc ccatcctggt cgagctggac      60

ggcgacgtaa acggccacag gttcagcgtg tccggcgagg gcgagggcga tgccacctac     120

ggcaagctga ccctgaagtt catctgcacc accggcaagc tgcccgtgcc ctggcccacc     180

ctcgtgacca ccctgacctg gggggtgcag tgcttcgccc gctaccccga ccacatgaag     240

cagcacgact tcttcaagtc cgccatgccc gaaggctacg tccaggagcg taccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacat cagccacaac gtctatatca ccgccgacaa gcagaagaac     480

ggcatcaagg cccacttcaa gatccgccac aacatcgagg acggcggcgt gcagctcgcc     540

gaccactacc agcagaacac ccccatcggc gacggccccg tgctgctgcc cgacaaccac     600

tacctgagca cccagtccaa gctgagcaaa gaccccaacg agaagcgcga tcacatggtc     660

ctgctggagt tcgtgaccgc cgccgggatc actctcggca tggacgagct gtacaagtaa     720

<210>  15
<211>  239
<212>  PRT
<213>  Artificial Sequence

<220>
<223>  ECFP-S72A-S175G-A206K

<400> 15

Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ala Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Gly
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Lys Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  16
<211>  720
<212>  DNA
<213>  Artificial Sequence

<220>
<223>  UMFP-1

<400>  16
atggtgagca agggcgagga gctgttcacc ggggtggtgc ccatcctggt cgagctggac      60

ggcgacgtaa acggccacag gttcagcgtg tccggcgagg gcgagggcga tgccacctac     120

ggcaagctga ccctgaagtt catctgcacc accggcaagc tgcccgtgcc ctggcccacc     180

ctcgtgacca ccctgacctt cggggtgcag tgcttcgccc gctaccccga ccacatgaag     240

cagcacgact tcttcaagtc cgccatgccc gaaggctacg tccaggagcg taccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacat cagccacaac gtctatatca ccgccgacaa gcagaagaac     480

ggcatcaagg cccacttcaa gatccgccac aacatcgagg acggcggcgt gcagctcgcc     540

gaccactacc agcagaacac ccccatcggc gacggccccg tgctgctgcc cgacaaccac     600

tacctgagca cccagtccaa gctgagcaaa gaccccaacg agaagcgcga tcacatggtc     660

ctgctggagt tcgtgaccgc cgccgggatc actctcggca tggacgagct gtacaagtaa     720

<210>  17
<211>  239
<212>  PRT
<213>  Artificial Sequence

<220>
<223>  UMFP-1

<400>  17

Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile

35                  40                  45

Cys Thr Thr Gly Lys Leu Pro Val Pro Trp Pro Thr Leu Val Thr Thr
    50                  55                  60

Leu Thr Phe Gly Val Gln Cys Phe Ala Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Gly
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Lys Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  18
<211>  720
<212>  DNA
<213>  Artificial Sequence

<220>
<223>  ECFP-W66F-S72A-S175G-A206K-T65Q-Y145G-H148S

<400> 18
atggtgagca agggcgagga gctgttcacc ggggtggtgc ccatcctggt cgagctggac 60

ggcgacgtaa acggccacag gttcagcgtg tccggcgagg gcgagggcga tgccacctac 120

ggcaagctga ccctgaagtt catctgcacc accggcaagc tgcccgtgcc ctggcccacc 180

ctcgtgacca ccctgcaatt cggggtgcag tgcttcgccc gctaccccga ccacatgaag 240

cagcacgact tcttcaagtc cgccatgccc gaaggctacg tccaggagcg taccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaacgggat aagctcaaac gtatatatca ccgccgacaa gcagaagaac 480

ggcatcaagg cccacttcaa gatccgccac aacatcgagg acggcggcgt gcagctcgcc 540

gaccactacc agcagaacac ccccatcggc gacggccccg tgctgctgcc cgacaaccac 600

tacctgagca cccagtccaa gctgagcaaa gaccccaacg agaagcgcga tcacatggtc 660

ctgctggagt tcgtgaccgc cgccgggatc actctcggca tggacgagct gtacaagtaa 720

<210> 19
<211> 239
<212> PRT
<213> Artificial Sequence

<220>
<223> ECFP-W66F-S72A-S175G-A206K-T65Q-Y145G-H148S

<400> 19

Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Gln Phe Gly Val Gln Cys Phe Ala Arg Tyr Pro Asp His Met Lys
65                  70                  75                  80

Gln His Asp Phe Phe Lys Ser Ala Met Pro Glu Gly Tyr Val Gln Glu

                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Gly Ile Ser Ser Asn Val Tyr Ile Thr Ala Asp Lys Gln Lys Asn
145                 150                 155                 160

Gly Ile Lys Ala His Phe Lys Ile Arg His Asn Ile Glu Asp Gly Gly
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Lys Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210> 20
<211> 720
<212> DNA
<213> Artificial Sequence

<220>
<223> UMFP-2

<400> 20
atggtgagca agggcgagga gctgttcacc ggggtggtgc ccatcctggt cgagctggac 60

ggcgacgtaa acggccacag gttcagcgtg tccggcgagg gcgagggcga tgccacctac 120

ggcaagctga ccctgaagct catctgcacc accggcaagc tgcccgtgcc ctggcccacc 180

ctcgtgacca ccctgcaatt cggggtgcag tgcttcgccc gctaccccga ccacatgaag 240

cagcacgact tcttcaagtc cgccatgccc gaaggctacg tccaggagcg taccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaacgggat aagctcaaac gtatatatca ccgccgacaa gcagaagaac 480

ggcatcaagg cccacttcaa gatccgccac aacatcgagg acggcggcgt gcagctcgcc 540

gaccactacc agcagaacac ccccatcggc gacggccccg tgctgctgcc cgacaaccac 600

tacctgagca cccagtccaa gctgagcaaa gaccccaacg agaagcgcga tcacatggtc 660

ctgctggagt tcgtgaccgc cgccgggatc actctcggca tggacgagct gtacaagtaa 720

<210> 21
<211> 239
<212> PRT
<213> Artificial Sequence

<220>
<223> UMFP-2

<400> 21

Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Leu Ile
        35                  40                  45

Cys Thr Thr Gly Lys Leu Pro Val Pro Trp Pro Thr Leu Val Thr Thr
    50                  55                  60

Leu Gln Phe Gly Val Gln Cys Phe Ala Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr

130                 135                 140

Asn Gly Ile Ser Ser Asn Val Tyr Ile Thr Ala Asp Lys Gln Lys Asn
145                 150                 155                 160

Gly Ile Lys Ala His Phe Lys Ile Arg His Asn Ile Glu Asp Gly Gly
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Lys Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  22
<211>  720
<212>  DNA
<213>  Artificial Sequence

<220>
<223>  UMFP-3

<400>  22
atggtgagca agggcgagga gctgttcacc ggggtggtgc ccatcctggt cgagctggac      60

ggcgacgtaa acggccacag gttcagcgtg tccggcgagg gcgagggcga tgccacctac     120

ggcaagctga ccctgaagct catctgcacc accggcaagc tgcccgtgcc ctggcccacc     180

ctcgtgacca ccctgcaatt cggggtgcag tgcttcgccc gctaccccga ccacatgaag     240

cagcacgact tcttcaagtc cgccatgccc gaaggctacg tccaggagcg taccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaacgggat aagctcaaac gtatatatca ccgccgacaa gcagaagaac     480

ggcatcaagg cccacttcaa gatccgccac aacatcgagg acggcggcgt gcagctcgcc     540

gaccactacc agcagaacac ccccatcggc gacggccccg tgctgctgcc cgacaaccac     600

tacctgagcg tccagtccaa gctgagcaaa gaccccaacg agaagcgcga tcacatggtc     660

ctgctggagt tcgtgaccgc cgccgggatc actctcggca tggacgagct gtacaagtaa     720

<210>  23
<211>  239
<212>  PRT
<213>  Artificial Sequence

<220>
<223>  UMFP-3

<400>  23

Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Leu Ile
        35                  40                  45

Cys Thr Thr Gly Lys Leu Pro Val Pro Trp Pro Thr Leu Val Thr Thr
    50                  55                  60

Leu Gln Phe Gly Val Gln Cys Phe Ala Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Gly Ile Ser Ser Asn Val Tyr Ile Thr Ala Asp Lys Gln Lys Asn
145                 150                 155                 160

Gly Ile Lys Ala His Phe Lys Ile Arg His Asn Ile Glu Asp Gly Gly
                165                 170                 175

Val Gln Leu Ala Asp His Tyr Gln Gln Asn Thr Pro Ile Gly Asp Gly

            180                 185                 190

Pro Val Leu Leu Pro Asp Asn His Tyr Leu Ser Val Gln Ser Lys Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210> 24
<211> 1395
<212> DNA
<213> Artificial Sequence

<220>
<223> C(delta)11UMFP-LE-N(delta)4ECFP

<400> 24
atggtgagca agggcgagga gctgttcacc ggggtggtgc ccatcctggt cgagctggac 60

ggcgacgtaa acggccacag gttcagcgtg tccggcgagg gcgagggcga tgccacctac 120

ggcaagctga ccctgaagct catctgcacc accggcaagc tgcccgtgcc ctggcccacc 180

ctcgtgacca ccctgcaatt cggggtgcag tgcttcgccc gctaccccga ccacatgaag 240

cagcacgact tcttcaagtc cgccatgccc gaaggctacg tccaggagcg taccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaacgggat aagctcaaac gtatatatca ccgccgacaa gcagaagaac 480

ggcatcaagg cccacttcaa gatccgccac aacatcgagg acggcggcgt gcagctcgcc 540

gaccactacc agcagaacac ccccatcggc gacggccccg tgctgctgcc cgacaaccac 600

tacctgagcg tccagtccaa gctgagcaaa gaccccaacg agaagcgcga tcacatggtc 660

ctgctggagt tcgtgaccgc cgccctcgag gaggagctgt tcaccggggt ggtgcccatc 720

ctggtcgagc tggacggcga cgtaaacggc cacaagttca gcgtgtccgg cgagggcgag 780

ggcgatgcca cctacggcaa gctgaccctg aagttcatct gcaccaccgg caagctgccc 840

gtgccctggc ccaccctcgt gaccaccctg acctggggcg tgcagtgctt cagccgctac 900

cccgaccaca tgaagcagca cgacttcttc aagtccgcca tgcccgaagg ctacgtccag 960

gagcgcacca tcttcttcaa ggacgacggc aactacaaga cccgcgccga ggtgaagttc 1020

gagggcgaca ccctggtgaa ccgcatcgag ctgaagggca tcgacttcaa ggaggacggc 1080

aacatcctgg ggcacaagct ggagtacaac tacatcagcc acaacgtcta tatcaccgcc 1140

gacaagcaga agaacggcat caaggccaac ttcaagatcc gccacaacat cgaggacggc 1200

agcgtgcagc tcgccgacca ctaccagcag aacaccccca tcggcgacgg ccccgtgctg 1260

ctgcccgaca accactacct gagcacccag tccgccctga gcaaagaccc caacgagaag 1320

cgcgatcaca tggtcctgct ggagttcgtg accgccgccg ggatcactct cggcatggac 1380

gagctgtaca agtaa 1395

<210> 25
<211> 464
<212> PRT
<213> Artificial Sequence

<220>
<223> C(delta)11UMFP-LE-N(delta)4ECFP

<400> 25

Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Leu Ile
        35                  40                  45

Cys Thr Thr Gly Lys Leu Pro Val Pro Trp Pro Thr Leu Val Thr Thr
    50                  55                  60

Leu Gln Phe Gly Val Gln Cys Phe Ala Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr

130                 135                 140

Asn Gly Ile Ser Ser Asn Val Tyr Ile Thr Ala Asp Lys Gln Lys Asn
145                 150                 155                 160

Gly Ile Lys Ala His Phe Lys Ile Arg His Asn Ile Glu Asp Gly Gly
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Val Gln Ser Lys Leu
        195                 200                 205

Ser Lys Asp Pro Asn Glu Lys Arg Asp His Met Val Leu Leu Glu Phe
    210                 215                 220

Val Thr Ala Ala Leu Glu Glu Glu Leu Phe Thr Gly Val Val Pro Ile
225                 230                 235                 240

Leu Val Glu Leu Asp Gly Asp Val Asn Gly His Lys Phe Ser Val Ser
                245                 250                 255

Gly Glu Gly Glu Gly Asp Ala Thr Tyr Gly Lys Leu Thr Leu Lys Phe
            260                 265                 270

Ile Cys Thr Thr Gly Lys Leu Pro Val Pro Trp Pro Thr Leu Val Thr
        275                 280                 285

Thr Leu Thr Trp Gly Val Gln Cys Phe Ser Arg Tyr Pro Asp His Met
    290                 295                 300

Lys Gln His Asp Phe Phe Lys Ser Ala Met Pro Glu Gly Tyr Val Gln
305                 310                 315                 320

Glu Arg Thr Ile Phe Phe Lys Asp Asp Gly Asn Tyr Lys Thr Arg Ala
                325                 330                 335

Glu Val Lys Phe Glu Gly Asp Thr Leu Val Asn Arg Ile Glu Leu Lys
            340                 345                 350

Gly Ile Asp Phe Lys Glu Asp Gly Asn Ile Leu Gly His Lys Leu Glu
        355                 360                 365

Tyr Asn Tyr Ile Ser His Asn Val Tyr Ile Thr Ala Asp Lys Gln Lys
    370                 375                 380

Asn Gly Ile Lys Ala Asn Phe Lys Ile Arg His Asn Ile Glu Asp Gly
385                 390                 395                 400

Ser Val Gln Leu Ala Asp His Tyr Gln Gln Asn Thr Pro Ile Gly Asp
                405                 410                 415

Gly Pro Val Leu Leu Pro Asp Asn His Tyr Leu Ser Thr Gln Ser Ala
            420                 425                 430

Leu Ser Lys Asp Pro Asn Glu Lys Arg Asp His Met Val Leu Leu Glu
        435                 440                 445

Phe Val Thr Ala Ala Gly Ile Thr Leu Gly Met Asp Glu Leu Tyr Lys
    450                 455                 460

<210>  26
<211>  1491
<212>  DNA
<213>  Artificial Sequence

<220>
<223>  UC-SCAT3

<400>  26
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgacctg gggcgtgcag tgcttcagcc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacat cagccacaac gtctatatca ccgccgacaa gcagaagaac     480

ggcatcaagg ccaacttcaa gatccgccac aacatcgagg acggcagcgt gcagctcgcc     540

gaccactacc agcagaacac ccccatcggc gacggccccg tgctgctgcc cgacaaccac     600

tacctgagca cccagtccgc cctgagcaaa gaccccaacg agaagcgcga tcacatggtc     660

ctgctggagt tcgtgaccgc cgccgggatc actctcggca tggacgagct gtacaagtcc     720

tcgtccgagc tcagcggaga tgaggtcgat ggtaccagcg gaagcgaatt catggtgagc 780

aagggcgagg agctgttcac cggggtggtg cccatcctgg tcgagctgga cggcgacgta 840

aacggccaca ggttcagcgt gtccggcgag ggcgagggcg atgccaccta cggcaagctg 900

accctgaagc tcatctgcac caccggcaag ctgcccgtgc cctggcccac cctcgtgacc 960

accctgcaat tcggggtgca gtgcttcgcc cgctaccccg accacatgaa gcagcacgac 1020

ttcttcaagt ccgccatgcc cgaaggctac gtccaggagc gtaccatctt cttcaaggac 1080

gacggcaact acaagacccg cgccgaggtg aagttcgagg gcgacaccct ggtgaaccgc 1140

atcgagctga agggcatcga cttcaaggag gacggcaaca tcctggggca caagctggag 1200

tacaacggga taagctcaaa cgtatatatc accgccgaca agcagaagaa cggcatcaag 1260

gcccacttca agatccgcca caacatcgag gacggcggcg tgcagctcgc cgaccactac 1320

cagcagaaca cccccatcgg cgacggcccc gtgctgctgc ccgacaacca ctacctgagc 1380

gtccagtcca agctgagcaa agaccccaac gagaagcgcg atcacatggt cctgctggag 1440

ttcgtgaccg ccgccgggat cactctcggc atggacgagc tgtacaagta a 1491

<210> 27
<211> 496
<212> PRT
<213> Artificial Sequence

<220>
<223> UC-SCAT3

<400> 27

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu

                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys Ser
225                 230                 235                 240

Ser Ser Glu Leu Ser Gly Asp Glu Val Asp Gly Thr Ser Gly Ser Glu
                245                 250                 255

Phe Met Val Ser Lys Gly Glu Glu Leu Phe Thr Gly Val Val Pro Ile
            260                 265                 270

Leu Val Glu Leu Asp Gly Asp Val Asn Gly His Arg Phe Ser Val Ser
        275                 280                 285

Gly Glu Gly Glu Gly Asp Ala Thr Tyr Gly Lys Leu Thr Leu Lys Leu
    290                 295                 300

Ile Cys Thr Thr Gly Lys Leu Pro Val Pro Trp Pro Thr Leu Val Thr
305                 310                 315                 320

Thr Leu Gln Phe Gly Val Gln Cys Phe Ala Arg Tyr Pro Asp His Met
                325                 330                 335

Lys Gln His Asp Phe Phe Lys Ser Ala Met Pro Glu Gly Tyr Val Gln
            340                 345                 350

Glu Arg Thr Ile Phe Phe Lys Asp Asp Gly Asn Tyr Lys Thr Arg Ala
        355                 360                 365

Glu Val Lys Phe Glu Gly Asp Thr Leu Val Asn Arg Ile Glu Leu Lys
    370                 375                 380

Gly Ile Asp Phe Lys Glu Asp Gly Asn Ile Leu Gly His Lys Leu Glu
385                 390                 395                 400

Tyr Asn Gly Ile Ser Ser Asn Val Tyr Ile Thr Ala Asp Lys Gln Lys
                405                 410                 415

Asn Gly Ile Lys Ala His Phe Lys Ile Arg His Asn Ile Glu Asp Gly
            420                 425                 430

Gly Val Gln Leu Ala Asp His Tyr Gln Gln Asn Thr Pro Ile Gly Asp
        435                 440                 445

Gly Pro Val Leu Leu Pro Asp Asn His Tyr Leu Ser Val Gln Ser Lys
    450                 455                 460

Leu Ser Lys Asp Pro Asn Glu Lys Arg Asp His Met Val Leu Leu Glu
465                 470                 475                 480

Phe Val Thr Ala Ala Gly Ile Thr Leu Gly Met Asp Glu Leu Tyr Lys
                485                 490                 495

<210>  28
<211>  720
<212>  DNA
<213>  Artificial Sequence

<220>
<223>  UMFP-4

<400>  28
atggtgagca agggcgagga gctgttcacc ggggtggtgc ccatcctggt cgagctggac      60

ggcgacgtaa acggccacag gttcagcgtg tccggcgagg gcgagggcga tgccacctac     120

ggcaagctga ccctgaagct catctgcacc accggcaagc tgcccgtgcc ctggcccacc 180

ctcgtgacca ccctgcaatt cggcgtgctg tgcttcgccc gctaccccga ccacatgaag 240

cagcacgact tcttcaagtc cgccatgccc gaaggctacg tccaggagcg taccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaacgggat aagctcaaac gtatatatca ccgccgacaa gcagaagaac 480

ggcatcaagg cccacttcaa gatccgccac aacatcgagg acggcggcgt gcagctcgcc 540

gaccactacc agcagaacac ccccatcggc gacggccccg tgctgctgcc cgacaaccac 600

tacctgagcg tccagtccaa gctgagcaaa gaccccaacg agaagcgcga tcacatggtc 660

ctgctggagt tcgtgaccgc cgccgggatc actctcggca tggacgagct gtacaagtaa 720

<210> 29
<211> 239
<212> PRT
<213> Artificial Sequence

<220>
<223> UMFP-4

<400> 29

Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Leu Ile
        35                  40                  45

Cys Thr Thr Gly Lys Leu Pro Val Pro Trp Pro Thr Leu Val Thr Thr
    50                  55                  60

Leu Gln Phe Gly Val Leu Cys Phe Ala Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Gly Ile Ser Ser Asn Val Tyr Ile Thr Ala Asp Lys Gln Lys Asn
145                 150                 155                 160

Gly Ile Lys Ala His Phe Lys Ile Arg His Asn Ile Glu Asp Gly Gly
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Val Gln Ser Lys Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  30
<211>  720
<212>  DNA
<213>  Artificial Sequence

<220>
<223>  ECFP-F46L-T65Q-W66F-Q69L-S72A-Y145G-H148S-S175G-A206K-T203V-F223S
       (Sirius)

<400>  30
atggtgagca agggcgagga gctgttcacc ggggtggtgc ccatcctggt cgagctggac      60

ggcgacgtaa acggccacag gttcagcgtg tccggcgagg gcgagggcga tgccacctac     120

ggcaagctga ccctgaagct catctgcacc accggcaagc tgcccgtgcc ctggcccacc     180

ctcgtgacca ccctgcaatt cggcgtgctg tgcttcgccc gctaccccga ccacatgaag     240

cagcacgact tcttcaagtc cgccatgccc gaaggctacg tccaggagcg taccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaacgggat aagctcaaac gtatatatca ccgccgacaa gcagaagaac     480

ggcatcaagg cccacttcaa gatccgccac aacatcgagg acggcggcgt gcagctcgcc 540

gaccactacc agcagaacac ccccatcggc gacggccccg tgctgctgcc cgacaaccac 600

tacctgagcg tccagtccaa gctgagcaaa gaccccaacg agaagcgcga tcacatggtc 660

ctgctggagt ccgtgaccgc cgccgggatc actctcggca tggacgagct gtacaagtaa 720

<210> 31
<211> 239
<212> PRT
<213> Artificial Sequence

<220>
<223> ECFP-F46L-T65Q-W66F-Q69L-S72A-Y145G-H148S-S175G-A206K-T203V-F223S
(Sirius)

<400> 31

Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Leu Ile
        35                  40                  45

Cys Thr Thr Gly Lys Leu Pro Val Pro Trp Pro Thr Leu Val Thr Thr
    50                  55                  60

Leu Gln Phe Gly Val Leu Cys Phe Ala Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Gly Ile Ser Ser Asn Val Tyr Ile Thr Ala Asp Lys Gln Lys Asn

145                 150                 155                 160

Gly Ile Lys Ala His Phe Lys Ile Arg His Asn Ile Glu Asp Gly Gly
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Val Gln Ser Lys Leu
        195                 200                 205

Ser Lys Asp Pro Asn Glu Lys Arg Asp His Met Val Leu Leu Glu Ser
    210                 215                 220

Val Thr Ala Ala Gly Ile Thr Leu Gly Met Asp Glu Leu Tyr Lys
225                 230                 235

<210>  32
<211>  1491
<212>  DNA
<213>  Artificial Sequence

<220>
<223>  SC-SCAT3

<400>  32
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgacctg gggcgtgcag tgcttcagcc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacat cagccacaac gtctatatca ccgccgacaa gcagaagaac     480

ggcatcaagg ccaacttcaa gatccgccac aacatcgagg acggcagcgt gcagctcgcc     540

gaccactacc agcagaacac ccccatcggc gacggccccg tgctgctgcc cgacaaccac     600

tacctgagca cccagtccgc cctgagcaaa gaccccaacg agaagcgcga tcacatggtc     660

ctgctggagt tcgtgaccgc cgccgggatc actctcggca tggacgagct gtacaagtcc     720

tcgtccgagc tcagcggaga tgaggtcgat ggtaccagcg gaagcgaatt catggtgagc     780

aagggcgagg agctgttcac cggggtggtg cccatcctgg tcgagctgga cggcgacgta 840

aacggccaca ggttcagcgt gtccggcgag ggcgagggcg atgccaccta cggcaagctg 900

accctgaagc tcatctgcac caccggcaag ctgcccgtgc cctggcccac cctcgtgacc 960

accctgcaat tcggcgtgct gtgcttcgcc cgctaccccg accacatgaa gcagcacgac 1020

ttcttcaagt ccgccatgcc cgaaggctac gtccaggagc gtaccatctt cttcaaggac 1080

gacggcaact acaagacccg cgccgaggtg aagttcgagg gcgacaccct ggtgaaccgc 1140

atcgagctga agggcatcga cttcaaggag gacggcaaca tcctggggca caagctggag 1200

tacaacggga taagctcaaa cgtatatatc accgccgaca agcagaagaa cggcatcaag 1260

gcccacttca agatccgcca caacatcgag gacggcggcg tgcagctcgc cgaccactac 1320

cagcagaaca cccccatcgg cgacggcccc gtgctgctgc ccgacaacca ctacctgagc 1380

gtccagtcca agctgagcaa agaccccaac gagaagcgcg atcacatggt cctgctggag 1440

tccgtgaccg ccgccgggat cactctcggc atggacgagc tgtacaagta a 1491

<210> 33
<211> 496
<212> PRT
<213> Artificial Sequence

<220>
<223> SC-SCAT3

<400> 33

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys Ser
225                 230                 235                 240

Ser Ser Glu Leu Ser Gly Asp Glu Val Asp Gly Thr Ser Gly Ser Glu
                245                 250                 255

Phe Met Val Ser Lys Gly Glu Glu Leu Phe Thr Gly Val Val Pro Ile
            260                 265                 270

Leu Val Glu Leu Asp Gly Asp Val Asn Gly His Arg Phe Ser Val Ser
        275                 280                 285

Gly Glu Gly Glu Gly Asp Ala Thr Tyr Gly Lys Leu Thr Leu Lys Leu
    290                 295                 300

Ile Cys Thr Thr Gly Lys Leu Pro Val Pro Trp Pro Thr Leu Val Thr
305                 310                 315                 320

Thr Leu Gln Phe Gly Val Leu Cys Phe Ala Arg Tyr Pro Asp His Met

                325                 330                 335

Lys Gln His Asp Phe Phe Lys Ser Ala Met Pro Glu Gly Tyr Val Gln
            340                 345                 350

Glu Arg Thr Ile Phe Phe Lys Asp Asp Gly Asn Tyr Lys Thr Arg Ala
        355                 360                 365

Glu Val Lys Phe Glu Gly Asp Thr Leu Val Asn Arg Ile Glu Leu Lys
    370                 375                 380

Gly Ile Asp Phe Lys Glu Asp Gly Asn Ile Leu Gly His Lys Leu Glu
385                 390                 395                 400

Tyr Asn Gly Ile Ser Ser Asn Val Tyr Ile Thr Ala Asp Lys Gln Lys
                405                 410                 415

Asn Gly Ile Lys Ala His Phe Lys Ile Arg His Asn Ile Glu Asp Gly
            420                 425                 430

Gly Val Gln Leu Ala Asp His Tyr Gln Gln Asn Thr Pro Ile Gly Asp
        435                 440                 445

Gly Pro Val Leu Leu Pro Asp Asn His Tyr Leu Ser Val Gln Ser Lys
    450                 455                 460

Leu Ser Lys Asp Pro Asn Glu Lys Arg Asp His Met Val Leu Leu Glu
465                 470                 475                 480

Ser Val Thr Ala Ala Gly Ile Thr Leu Gly Met Asp Glu Leu Tyr Lys
                485                 490                 495

## Description

### FIELD OF THE INVENTION

The present invention relates to a fluorescent protein having improved fluorescence properties and, more specifically, to a fluorescent protein that fluoresces an ultramarine color.

### BACKGROUND ART

A fluorescent protein, so-called GFP (green fluorescence protein) derived from Aequorea victoria, which is one of bioluminescent jellyfish, has an excitation peak at 395 nm and a maximum emission at 509 nm and emits a green color (Chalfie et al., Science, 1994, 263, 802-805). This protein has advantages that is stable at high temperatures (Tm = 78°C), stable in chaotropic reagents (e.g., 8M urea), relatively stably expressed as a fusion protein with other protein so that the presence of the fusion protein can be visually recognized by its fluorescence emission, and the like. This enables to observe and confirm the localization of a specific substance in living organisms or cells and further enables to confirm the expression of a specific gene, using intact living organisms or cells, resulting in a major breakthrough in studies of molecular biology.

Investigations have been intensively carried out to further improve the utility of this fluorescent protein, and many reports have been presented on artificial mutants where a specific amino acid residue(s) of GFP is/are substituted with other amino acid residue(s). The purpose to produce artificial mutants of GFP is broadly divided into increase in fluorescence intensity and shift in emission spectra. In particular, it becomes possible to concurrently confirm the localization of a plurality of different substances or the expression of a plurality of genes by using a plurality of fluorescent proteins having shifted emission spectra. Thus, mutants that produce various fluorescence colors have been reported.

The mutation sites in artificial mutants of GFP and their characteristics reported so far are, for example, as follows, in which notations for the artificial mutants of GFP are used to denote, e.g., as Y66H a mutant wherein the 66th amino acid residue tyrosine (Y, expressed by one-letter code for amino acids unless otherwise indicated) from the N terminus in the amino acid sequence for wild type GFP is substituted with H, and a mutant wherein a plurality of amino acid residues are concurrently substituted is expressed by connecting the respective substitutions with hyphen (-).

Y66H: a fluorescent protein emitting blue fluorescence with lower fluorescence intensities; the fluorescence disappears rapidly (Non-Patent Literature 1).

V163A: a fluorescent protein emitting blue fluorescence; V163A-S175G acquires heat resistance to provide enhanced fluorescence intensities (Patent Literature 1).

F64I, F64V, F64A, F64G, F64L: fluorescent proteins with the same emission wavelength but enhanced fluorescence intensities (Patent Literature 2).

F64L-S65T-Y6bH-Y145F: fluorescent proteins emitting blue fluorescence with lower fluorescence intensities; the fluorescence disappears rapidly (Patent Literature 3).

F64L-Y66H-YI45F-L236R, F64L-Y66H-Y145F-V163A-S 175G-L236R, Y66H-Y145F-V163A-S175G, F64L-Y66H-Y145F: fluorescent proteins having photostability (Patent Literature 4)

F64L-Y66H-S 175G: blue fluorescent protein having stable fluorescence properties and having different excitation spectra and/or emission spectra (Patent Literature 5)

F64L-Y66H-V163A: blue fluorescent protein having more enhanced fluorescence intensities (Patent Literature 6)
[Non-Patent Literature 1] Heim et al., 1994, Proc. Natl. Acad. Sci. USA, 91, 12501-12504
[Patent Literature 1] WO 96/27675
[Patent Literature 2] USP 6,172.188
[Patent Literature 3] USP 5,777,079
[Patent Literature 4] USP 6,194,548
[Patent Literature 5] Japanese National Publication (Tokuhyo) No. 2005-511027
[Patent Literature 6] Japanese National Publication (Tokuhyo) No. 2000-509987

### SUMMARY OF THE INVENTION

### TECHNICAL PROBLEM

A first object of the present invention is to provide a novel fluorescent protein having a new maximum emission peak, which has not been reported so far. A second object of the present invention is to provide a previously unreported, novel fluorescent protein with new emission spectra having pH-independent fluorescence intensities where the fluorescence intensities are not affected by pH changes, since fluorescence intensities of known fluorescent protein mutants including wild type GFP depend greatly upon changes in pH and the fluorescence is almost lost under acidic conditions.

### SOLUTION TO PROBLEM

The present inventors have conducted investigations to construct artificial mutants of GFP having previously unreported, novel emission peaks, especially having fluorescence intensities which are stably maintained over a wide range of pH, and found that mutants acquired by substitution of specific amino acids in GFP exhibit such properties. As a result, the inventions described below have been completed.
(1) A fluorescent protein having an emission peak at 424 nm, comprising an amino acid sequence represented by SEQ ID NO: 1, in which each of the amino acid residues at the 66th position and the 175th position is substituted and at least one of the amino acid residues at the 72nd position and the 206th position is further substituted.
(2) The fluorescent protein according to (1), wherein both of the amino acid residues at the 72nd position and the 206th position are substituted.
(3) The fluorescent protein according to (2), wherein the amino acid residue at the 66th position is substituted with phenylalanine, the amino acid residue at the 72nd position with alanine, the amino acid residue at the 175th position with glycine and the amino acid residue at the 206th position with lysine, respectively.
(4) The fluorescent protein according to any one of (1) to (3), wherein at least one of the amino acid residues at the 65th position, the 145th position and the 148th position is further substituted.
(5) The fluorescent protein according to (4), wherein all of the amino acid residues at the 65th position, the 145th position and the 148th position are substituted.
(6) The fluorescent protein according to (5), wherein the amino acid residue at the 65th position is substituted with glutamine, the amino acid residue at the 145th position with glycine and the amino acid residue at the 148th position with serine.
(7) The fluorescent protein according to any one of (4) to (6), wherein the amino acid residue at the 46th position is further substituted.
(8) The fluorescent protein according to (7), wherein the amino acid residue at the 46th position is substituted with leucine.
(9) The fluorescent protein according to any one of (1) to (8), wherein the amino acid residue at the 203rd position is further substituted.
(10) The fluorescent protein according to (9), wherein the amino acid residue at the 203rd position is substituted with valine.
(11) The fluorescent protein according to (10), wherein the amino acid residue at the 66th position is substituted with phenylalanine, the amino acid residue at the 175th position with glycine, the amino acid residue at the 72nd position with alanine, the amino acid residue at the 206th position with lysine, the amino acid residue at the 65th position with glutamine, the amino acid residue at the 145th position with glycine, the amino acid residue at the 148th position with serine, the amino acid residue at the 46th position with leucine, and the amino acid residue at the 203rd position with valine.
(12) The fluorescent protein having an emission peak at 424 nm according to (1) to (11), wherein one or more amino acids are deleted, substituted or added in the amino acid sequence.
(13) A fused protein comprising the fluorescent protein according to any one of (1) to (12) and an optional protein or polypeptide.
(14) A nucleic acid encoding the fluorescent protein or fused protein according to any one of (1) to (13).
(15) A vector capable of expressing a fluorescent protein or fused protein encoded by the nucleic acid according to (14).
(16) A host cell transformed or transfected with the expression vector according to (15).

### ADVANTAGEOUS EFFECT OF INVENTION

First, the fluorescent protein of the present invention emits fluorescence having an emission peak at 424 nm unknown heretofore and can be visually distinguished by its ultramarine color from other fluorescent proteins. Furthermore, the fluorescent protein of the invention having pH-independent fluorescence intensities, which are not affected by pH changes, enables to use the fluorescent protein in an acidic environment proved to be difficult so far.

### BRIEF DESCRIPTION OF THE DRAWINGS

FIG. 1 shows the fluorescent coloration of GFP, known amino acid-substituted mutants of GFP and UMFP-1 of the present invention.
FIG. 2 shows the absorption spectra and emission spectra of the fluorescent protein of the invention and known fluorescent proteins: the left side denotes the absorption spectra and the right side denotes the emission spectra.
FIG. 3 shows pH titration curves related to emission intensities of the fluorescent protein of the invention and known fluorescent protein mutants (GFP and BFP).
FIG. 4 shows the fluorescence attenuation curves obtained when UMFP-3 (red) and EBFP (blue) were excited by excitation light at 355 nm.
FIG. 5 shows the fluorescence spectra of CΔ11UMFP-LE-NΔ4ECFP (blue), UMFP-3 (red) and ECFP (cyan) when excited with excitation light at 355 nm.
FIG. 6 shows monitoring of caspase-3 activation in living cells using UC-SCAT3. The higher the ordinate, the more caspase-3 is activated. The abscissa denotes the time lapsed after the treatment with TNFα.
FIG. 7 shows the images of Escherichia coli expressing Sirius (sky blue) or EGFP (green) that is incorporated into Dictyostelium discoideum (differential interference images) and digested via phagocytosis. The numerical figures above the panels denote time (second) after Escherichia coli is incorporated into Dictyostelium discoideum.
FIG. 8 shows the dual imaging of caspase-3 activation and Ca²⁺ kinetics in living cells, using SC-SCAT3 and SapRC2, indicating that the warmer the color, the higher the activity of caspase-3 and the concentration of Ca²⁺. The numerical values above the upper column denote the time lapsed after the addition of TNFα.

### DESCRIPTIION OF THE PREFERRED EMBODIMENT

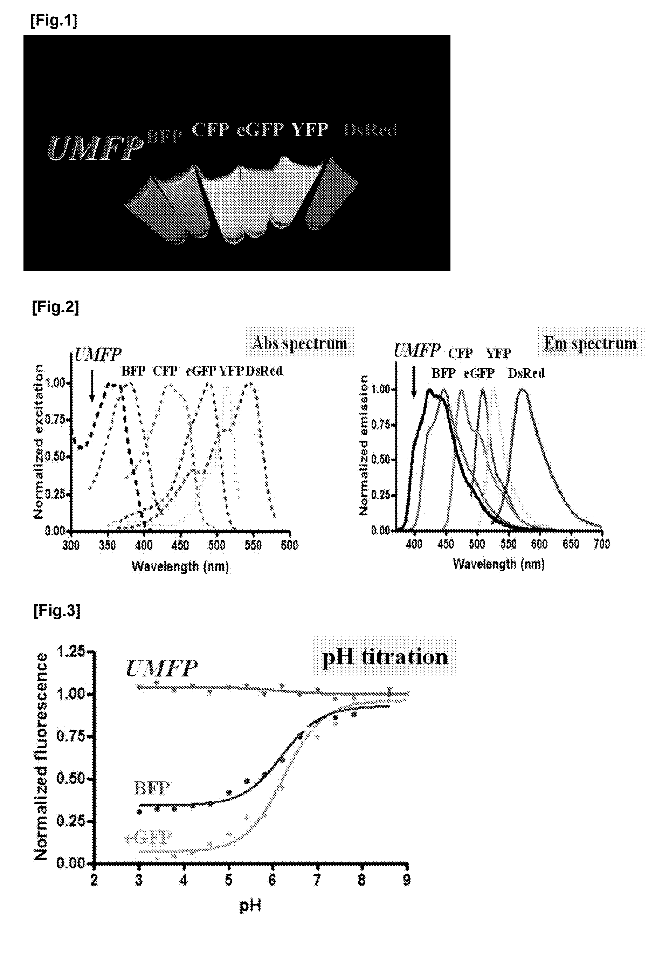

The present invention relates to the mutants of a fluorescent protein generally termed GFP. The mutants of the present invention include a fluorescent protein having an emission peak at 424 nm and comprising an amino acid sequence in which a specific amino acid residue(s) are substituted in the GFP from crystal jellyfish (genus Aequorea), e.g., Aequorea victoria, and a fluorescent protein having an emission peak at 424 nm and comprising an amino acid sequence in which one or more amino acids are further deleted, substituted or added. This emission wavelength is visually recognized as ultramarine to the naked eye, which can be visually distinguished clearly from the fluorescence color of green fluorescent protein known heretofore (FIG. 1). Hereinafter the protein of the present invention that emits ultramarine fluorescence is referred to as UMFP (Ultra Marine Fluorescence Protein).

The UMFP of the present invention is the fluorescent protein comprising the amino acid sequence represented by SEQ ID NO: 1 in which an amino acid(s) are substituted at the specific position(s). The amino acid sequence represented by SEQ ID NO: 1 is the amino acid sequence of wild type GFP from Aequorea victoria, in which F at the 64th position is substituted with L, S at the 65th position with T, Y at the 66th position with W, N at the 146th position with I, M at the 153rd position with T, V at the 163rd position with A and H at the 231 st position with L, respectively. Accordingly, the present invention is directed to the fluorescent protein having multiple substitution mutations in which amino acids are further substituted at specific positions of the amino acid sequence having substitution mutations at the 7 positions described above in the amino acid sequence of wild-type GFP. The amino acid sequence represented by SEQ ID NO: 1 was commercially available from Clontech, Inc. previously under the name of pECFP Vector (Catalog No. 632309).

The UMFP of the present invention is the protein comprising the amino acid sequence represented by SEQ ID NO: 1, in which said protein has substitutions of the amino acid residues at the 66th position (W) and the 175th position (S) and at least one substitution of the amino acid residues at the 72nd position (S) and the 206th position (A). This UMFP is referred to as UMFP-1 hereinafter. Preferably, UMFP-1 is a protein comprising the amino acid sequence represented by SEQ ID NO: 1, in which said protein has substitutions of 4 amino acid residues at the 66th, 175th, 72nd and 206th positions. Furthermore, UMFP-1 is preferably a fluorescent protein having subsitutions of the amino acid residue at the 66th position with F, the amino acid residue at the 72nd position with A, the amino acid residue at the 175th position with G and the amino acid residue at the 206th position with K, respectively. In describing the fluorescent protein of the present invention in terms of the substitution position and the kind of amino acid after substitution, the protein is expressed hereinafter by adding ECFP to the top of the hyphenated substitution position and amino acid after substitution, in order to indicate that the name is based on the amino acid sequence represented by SEQ ID NO: 1. For example, the preferred example of UMFP-1 described above is expressed as ECFP-W66F-S72A-S175G-A206K.

The present invention further includes the fluorescent protein in which any one of the amino acid residues at the 65th (T), 145th (Y) and 148th (H) positions in the UMFP-1 above is substituted or preferably all of these 3 amino acid residues are substituted concurrently. The UMFP containing additional substitution is referred to as UMFP-2 hereinafter. Preferably, the UMFP-2 is the fluorescent protein in which 3 amino acid residues at the 65th, 145th and 148th positions are further substituted. It is particularly preferred that UMFP-2 is the fluorescent protein in which the amino acid residue at the 65th position is substituted with Q, the amino acid residue at the 145th position with G and the amino acid residue at the 148th position with S, respectively. A particularly preferred example of the UMFP-2 of the present invention that the 3 amino acid residues are substituted with appropriate amino acid residues, respectively, is expressed by ECFP-W66F-S72A-S175G-A206K-T65Q-Y145G-H148S. UMFP-2 has its emission peak at 424 nm and further has an enhanced fluorescence intensity than UMFP-1. This fluorescence intensity can be more enhanced by further introducing an additional substitution of the amino acid residue at the 46th position (F), preferably the substitution referred to as F46L. The UMFP containing this substitution at the 46th position, i.e., ECFP-F46L-W66F-S72A-S175G-A206K-T65Q-Y145G-H148S is one of UMFP-2.

The present invention further includes the fluorescent protein in which the amino acid residue at the 203rd position (T) is further substituted in the UMFP-1 or UMFP-2 described above. The UMFP containing this additional substitution at the 203rd position is referred to as UMFP-3 hereinafter. A preferred substitution regarding UMFP-3 is T203V

The fluorescence wavelength of the protein of the invention can be measured preferably by optical means, for example, using a spectrophotometer, a fluorometer, a CCD image sensor, etc. Spectral characteristics can be measured in terms of the excitation wavelength properties and emission wavelength properties of the fluorescence emission by the protein of the present invention, and from these spectral characteristics, the respective peak wavelengths for the excitation wavelengths and emission wavelengths can be identified. The designation, e.g., "424 nm" is used in the present invention to mean preferably 424 ± 3 nm (more preferably 424 ± 2 nm), unless otherwise indicated.

By the amino acid substitution described above, the fluorescent protein of the present invention has emission peaks at about 424 nm or below, which is clearly distinct from known fluorescent proteins. For example, known fluorescent proteins have emission peaks at about 450 nm (e.g., BFP), about 470 nm (e.g., CFP), about 510 nm (e.g., eGFP), about 530 nm (e.g., YFP), about 600 nm (e.g., DsRed), etc. The emission peaks clearly different from these known emission peaks can provide fluorescence having different colors (e.g., ultramarine color in the present invention), which enables visual recognition to the naked eye (FIG. 1).

The fluorescent protein of the present invention has the excitation wavelength peak at approximately 355 nm, which is clearly different from known fluorescent proteins. For example, known fluorescent proteins have the excitation wavelength peak at about 380 nm (e.g., BFP), about 430 nm (e.g., CFP), about 480 nm (e.g., eGFP), about 510 nm (e.g., YFP), about 550 nm (e.g., DsRed), etc. Therefore, it is possible to emit the excitation light at wavelengths with which known fluorescent proteins can hardly react.

The fluorescent protein of the present invention, e.g., UMFP-3, has advantages that the protein has the emission peak at 424 nm and further its fluorescence intensities are maintained to a high level even under acidic conditions. The fluorescence intensities of conventional GFPs including wtGFP and the like are markedly reduced under acidic conditions, for example, under pH 5 or less, when compared to the fluorescence intensities under neutral to weakly alkaline conditions, e.g., at pH 7 to pH 9 (normally reduced by 70% to 100%), whereas the fluorescence intensities of the UMFP-3 of the invention under acidic conditions are maintained by at least 50%, preferably 75% or more and more preferably 90% or more, based on the fluorescence intensities under neutral to weakly alkaline conditions. In addition, the fluorescence intensities of the fluorescent protein of the present invention under acidic conditions (preferably at pH 5 or less and more preferably at pH 3 to pH 5) may be more intense than the fluorescence intensities under alkaline conditions (preferably at pH 7 or higher and more preferably at pH 7 to pH 9). That is, in the fluorescent protein of the present invention, the relative fluorescence intensity normalized to the fluorescence intensity at pH 9 can vary preferably within 50%, more preferably within 25% and most preferably within 10% in the pH range of, e.g., 3 to 9. The fluorescence intensity as described above which varies preferably within 50%, more preferably within 25% and most preferably within 10%, for the pH changes is referred to as pH-independent fluorescence intensity in the specification.

As used herein, the term "enhanced fluorescence intensity" means that the fluorescence level per mole of the fluorescent protein of the present invention for a given amount of excitation light having a certain wavelength is higher than that of conventional fluorescent proteins. An example of the enhanced fluorescence intensity by introducing mutation in the amino acid sequence of a fluorescent protein includes a comparison between the UMFP-1 and UMFP-3 of the present invention. In this example, when the excitation light at 355 nm is irradiated to each fluorescent protein, UMFP-3 provides more enhanced intensities by at least 20%, preferably by at least 30% and more preferably by at least 40%, than the intact UMFP-1. In addition, the fluorescent protein of the present invention has the excitation light shifted to a lower wavelength side as compared to known fluorescent proteins and can provide a relatively high fluorescence intensity under excitation light at lower wavelengths.

For example, the UMFP-3 where the T203V substitution is further introduced into ECFP-F46L-W66F-S72A-S175G-A206K-T65Q-Y145G-H148S which is one of UMFP-2, i.e., ECFP-F46L-W66F-S72A-S175G-A206K-T65Q-Y145V-H148S-T203V has the emission peak at 424 nm and has properties of an enhanced fluorescence intensity by at least 20%, preferably by at least 30% and more preferably by at least 40%, as compared to UMFP-1, and provides no significant change in its fluorescence intensity even under acidic conditions (for example, the change in fluorescence intensity is within 50%, preferably within 25% and more preferably within 10%).

The present invention further includes fluorescent proteins having the properties of UMFP described above, for example, having an emission peak at about 424 nm, preferably having an emission peak at about 424 nm and an enhanced fluorescence intensity, and more preferably having an emission peak at about 424 nm, an enhanced fluorescence intensity and a pH-independent fluorescence intensity, and comprising the amino acid sequence in which one or more amino acids are deleted, substituted or added at positions other than the mutation positions characteristic of the UMFP of the present invention, i.e., the 46th, 66th, 72nd, 175th, 206th, 65th, 145th, 148th and 203rd positions. In the substitution, deletion and/or addition of amino acids in the present invention, the term "one or more"' is used to mean variations of one to several ten amino acid residues, preferably 1 to 70, more preferably 1 to 50, much more preferably 1 to 30, particularly preferably 1 to 15, 1 to 14, 1 to 13, 1 to 12, 1 to 11, 1 to 10. 1 to 9, 1 to 8, 1 to 7, 1 to 6. 1 to 5, 1 to 4, 1 to 3, 1 to 2, or 1. The identity (%) of the amino acid sequence can be expressed as an amino acid sequence having the identity of at least 80%, preferably at least 85%, more preferably at least 90% and particularly preferably at least 95%, with the amino acid sequence represented by SEQ ID NO: 1.

It is empirically established that when physicochemical properties such as charges, size, hydrophobicity, etc. of amino acid residues are highly conserved mutations, such mutations are allowable for the amino acid sequence of a protein. Examples of the substitution of amino acid residues include glycine (Gly) and proline (Pro), Gly and alanine (Ala) or valine (Val), leucine (Leu) and isoleucine (Ile), glutamic acid (Glu) and glutamine (Gln), aspartic acid (Asp) and asparagine (Asn), cysteine (Cys) and threonine (Thr), Thr and serine (Ser) or Ala, lysine (Lys) and arginine (Arg), etc. Even beyond the conservation described above, a person skilled in the art will experience that any variation in which the essential function of the protein is not lost still remains. Accordingly, even in a protein comprising an amino acid sequence with a substitution, deletion, and/or addition of one or more amino acids in the amino acid sequence of SEQ ID NO: 1 at positions other than the specified positions for substitution in UMFP, i.e., the 46th position, the 66th position, the 72nd position, the 175th position, the 206th position, the 65th position, the 145th position, the 148th position and the 203rd position, some UMFPs may have the properties described above and it is understood that these proteins are also included as one embodiment of the present invention. For example, fluorescent proteins composed of the same amino acids as in the amino acid sequence of wtGFP except for the amino acids at positions other than the 46th, 66th, 72nd, 175th, 206th, 65th, 145th, 148th and 203rd positions described above are also included. Furthermore, the amino acid substitutions at positions other than the 46th, 66th, 72nd, 175th, 206th, 65th, 145th, 148th and 203rd positions described above may result in advantageous changes in properties such as improved enzyme stability, increased fluorescence intensity, etc., without damaging the functions characteristic of the UMFP of the present invention described above, and such fluorescent proteins are also included in the present invention.

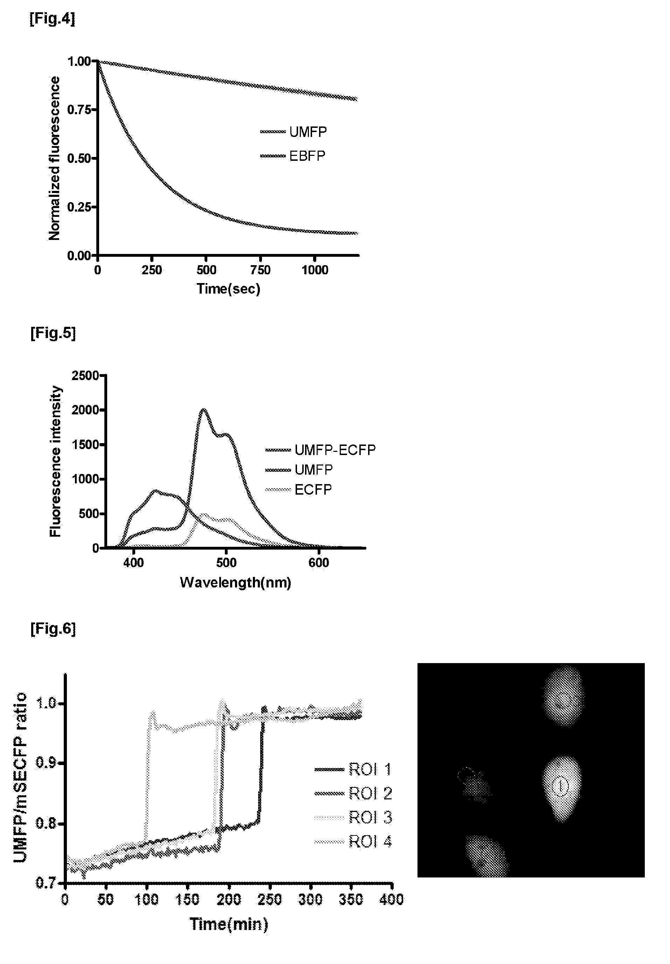

In addition, the protein of the present invention is advantageous also for fluorescence attenuation with lapse of time. That is, the fluorescent protein of the invention provides a prolonged attenuation time as compared to known fluorescent proteins. For example, as shown in EXAMPLE 6 and FIG. 4 in the specification, when the emission intensity after 1000 seconds to the emission intensity immediately after pulse irradiation for 10 seconds was measured, the fluorescent protein of the invention maintained approximately 80% of the emission intensity, whereas known EBFP maintained only approximately 10%. The fluorescent protein of the present invention is further characterized by providing linear fluorescence attenuation at least within 1000 seconds after irradiation.

The protein of the present invention may be produced and used alone, or may also be produced and used as a so-called fusion protein in which a protein(s) or polypeptide(s) other than the protein of the present invention is/are added to the protein of the invention at the N terminus and/or C terminus. Such fusion proteins comprising the protein of the present invention are one embodiment of the invention. In particular, the UMFP of the present invention can be used for the same purpose of using known GFP or its mutants, in place of them. For instance, the fusion protein of a certain protein and the UMFP of the present invention can be expressed in vivo or intracellularly to examine the in vivo or intracellular localization of the protein. Furthermore, the expression of the UMFP of the invention can be used as an index to examine the regulation system of gene expression in vivo or in cells. In particular, UMFP-3 of the invention has a pH-independent fluorescence intensity and can be used to confirm the localization of a protein in the acidic organelles, such as endosomes or lysosomes, where conventionally known GFPs or its mutants cannot be used or are found to be extremely difficult to apply, to observe behaviors of the protein in intracellular membranes, or to use the protein for different purposes.

The fluorescent protein of the present invention has the emission peak and excitation peak which are different from each peak possessed by known fluorescent proteins, and thus can be utilized for the case using a plurality of fluorescent proteins at the same time. For example, the light emission from the fluorescent protein of the invention (or its fusion proteins) can be visually distinguished from the light emission by known fluorescent proteins (or fusion proteins thereof) to the naked eye, because the emission peaks of the fluorescent protein of the invention are different from those of known fluorescent proteins (FIG. 1). In addition, the excitation peaks of the fluorescent protein of the invention are different from those of known fluorescent proteins, which makes it possible to construct the measurement system using wavelengths incapable of exciting known fluorescent proteins. Further by the advantages described above, the fluorescent protein of the invention and known fluorescent proteins can be used at the same time, and a concurrent multiple analysis which is more complicated and/or more excellent in distinctiveness than before can be performed.

Furthermore, the fluorescent protein of the present invention has different excitation and emission peaks from those of known fluorescent proteins as described above. Accordingly, by a suitable combination of the protein of the invention or its fusion protein and a known fluorescent protein, it is possible to construct a system using FRET (fluorescent resonance energy transfer) at wavelengths different from the wavelengths previously used. FRET and its application examples are known to those skilled in the art and described in, e.g., Takemoto, K., Nagai, T., Miyawaki, A. & Miura, M. Spatio-temporal activation of caspase revealed by indicator that is insensitive to environmental effects. J. Cell. Biol. 160, 235-243 (2003); Mizuno, H., Sawano, A., Eli, P., Hama, H. & Miyawaki, A. Red fluorescent protein from Discosoma as a fusion tag and a partner for fluorescence resonance energy transfer. Biochemistry. 40, 2502-2510 (2001), etc.

The fusion protein containing the protein of the present invention has an improved utility in that the function possessed by the functional protein is added, when compared to the case of producing or using the protein of the invention alone. Examples of such functional proteins include glutathione S-transferase (GST), maltose-bound protein (MBP), protein A and other proteins widely available for the production of fusion proteins. The protein of the invention can also be more advantageously produced by using functional polypeptides such as a FLAG tag, a histidine tag or a chitin-binding sequence that facilitate the production of recombinant proteins, especially the purification of recombinant proteins.

Depending upon necessity, an appropriate labeling compound such as a fluorescent substance, a radioactive substance, etc. can also be added to, or various chemical modifiers or high molecular weight materials such as polyethylene glycol can be bound to, the protein of the present invention or the fusion protein containing the protein of the present invention. In addition, the protein used in the present invention may also be bound to insoluble carriers. Chemical modifications targeted for these proteins are widely known to those skilled in the art, and may be applied to or used for the protein of the present invention modified in any way, as long as the functions of the protein of the present invention are not impaired.

Proteins can be exposed to various reaction conditions for the extraction operation, purification operation, etc. particularly in production of fusion proteins, or for the addition of a labeling compound in production of labeled proteins. The fluorescent protein of the present invention can maintain its activity pH-independently and is more tolerant under various reaction conditions than known fluorescent proteins. It is considered that the use of the protein of the invention is promoted by these properties.

The present invention provides the nucleic acid encoding the protein of the invention or the fusion protein containing the protein of the invention. The nucleic acid contains RNA or DNA and its form includes, but not particularly limited to, mRNA, cDNA, chemically synthesized DNA, etc. A preferred example of the nucleic acid is DNA. The nucleic acid of the present invention may be a single strand or may form a double strand or triple strand by base-pairing with a complementary nucleic acid or RNA to the sequence of the nucleic acid of the invention. The nucleic acid may also be labeled with an enzyme such as horse radish peroxidase (HRPO), etc., a radioactive isotope, a fluorescent material, a chemiluminescent material, or the like.

The nucleic acid of the present invention, preferably DNA encoding UMFP which is the protein of the present invention, comprises the nucleotide sequence encoding GFP represented by SEQ ID NO: 2, in which codons at positions other than the substitution positions characteristic of the UMFPs 1 to 3, i.e., the 46th, 66th, 72nd, 175th, 206th, 65th, 145th, 148th and 203rd positions described above, are substituted with the respective codons for the amino acid substitutions. A nucleic acid which hybridizes under stringent conditions to a nucleic acid comprising a complementary nucleotide sequence to the nucleotide sequence above and encodes the UMFP of the present invention is also included in the nucleic acid of the present invention. The term "stringent conditions" in the present invention refers to conditions that the nucleic acid hybridizes to a nucleic acid comprising a complementary nucleotide sequence to represented by SEQ ID NO: 2 in a buffer of 1.5 M salt concentration at 65°C and the hybridization is maintained under conditions of washing DNA in a 2 x SSC solution (containing 0.1% [w/v] SDS) at 50°C (1 x SSC: 0.15M NaCl and 0.015M sodium citrate). In terms of the identity (%) of the nucleotide sequence, it may be a nucleic acid comprising a nucleotide sequence having the identity of at least 70%, preferably at least 80%, more preferably at least 90% and particularly preferably at least 95%, with the nucleotide sequence represented by SEQ ID NO: 2. For the identity determination, the methods described in, e.g., Molecular Cloning 3rd Ed., Current Protocols in Molecular Biology, John Wiley & Sons 1987-1997, etc. can be used.

The nucleic acid of the present invention can be prepared by PCR, site-specific mutagenesis or other general genetic engineering techniques, based on DNA encoding the amino acid sequence represented by SEQ ID NO: 1, specifically, the DNA comprising the nucleotide sequence represented by SEQ ID NO: 2. The DNA comprising the nucleotide sequence represented by SEQ ID NO: 2 can be prepared by using vectors bearing the same, since these various vectors bearing the DNA are commercially available. Genetic engineering techniques including site-specific mutagenesis, etc. are described in, e.g., Maniatis T. et al. (Molecular Cloning, a Laboratory Manual, Cold Spring Harbor Laboratory, New York, 1982) and other manuals of experimental operations widely used for those skilled in the art. The nucleic acid of the present invention can also be produced by chemical synthesis such as the phosphoramidite method or using a commercially available DNA synthesizer, based on information of the nucleotide sequence represented by SEQ ID NO:2.

The DNA which is the nucleic acid encoding the protein of the present invention or the fusion protein containing the protein of the invention can be incorporated into an appropriate expression vector, and resulting expression vectors may be used for the production of the protein of the present invention or the fusion protein containing the protein of the invention by recombination. Such recombinant vectors for the production of the protein are also included in the present invention. The vectors of the invention may be in any form such as a circular form and a linear form. In addition to the nucleic acid encoding the protein of the present invention or the fusion protein containing the protein of the invention, the vectors may have any other nucleotide sequence, if necessary. Examples of the other nucleotide sequence include enhancer sequences, promoter sequences, ribosome binding sequences, nucleotide sequences for use in amplifying the number of copies, signal peptide-encoding nucleotide sequences, nucleotide sequences encoding any other polypeptide, poly A addition sequences, splicing sequences, replication origins, nucleotide sequences for genes as selection markers, and the like.

In genetic recombination, any appropriate synthetic DNA adaptor may be used for adding a translation initiation codon or a translation termination codon to the nucleic acid encoding the protein of the present invention or the fusion protein containing the protein of the invention, or for newly producing or deleting an appropriate restriction enzyme cleavage sequence in the nucleotide sequence. These operations fall within the routine work that those skilled in the art usually perform, and they can readily and optionally modify the nucleic acid encoding the protein of the present invention or the fusion protein containing the protein of the invention.

Any appropriate vector may be selected and used as the vector bearing the nucleic acid encoding the protein of the present invention or the fusion protein containing the protein of the invention, depending upon the host to be used. A variety of viruses such as bacteriophages, baculoviruses, retroviruses, vaccinia viruses, etc. may also be used, in addition to plasmids.

Commercially available expression vectors which can be used include, for example, pcDM8 (manufactured by Funakoshi Co.), pcDNAI (manufactured by Funakoshi Co.), pcDNAI/AmP (manufactured by Invitrogen Corp.), EGFP-C1 (manufactured by Clontech, Inc.), pREP4 (manufactured by Invitrogen Corp.), pGBT-9 (manufactured by Clontech, Inc.), etc. The protein of the present invention or the fusion protein containing the protein of the invention may be expressed under the control of a promoter sequence specific to the gene. Alternatively, other appropriate expression promoter may be linked upstream the nucleotide sequence encoding the protein of the present invention or the fusion protein containing the protein of the invention to provide for use. Such an expression promoter may be appropriately selected, depending on the host and the purpose of the expression. Examples of the promoter include, but are not limited to, a T7 promoter, a lac promoter, a trp promoter, a λPL promoter and the like, for an E. coli host; a PHO5 promoter, a GAP promoter, an ADH promoter, and the like, for a yeast host; and an SV40-derived promoter, a retrovirus promoter, a promoter for cytomegalovirus (human CMV) IE (immediate early) gene, a metallothionein promoter, a heat shock promoter, a SRα promoter, and the like, for an animal cell host. The operations for linking of the nucleic acid, preferably DNA, encoding the protein of the present invention or the fusion protein containing the protein of the invention to the promoters exemplified above or for its incorporation into the expression vector, or other operations can be performed in accordance with the descriptions given by Maniatis, et al. and other manuals of experimental operations.

The protein of the present invention or the fusion protein containing the protein of the invention may also be prepared by organic chemical synthesis, e.g., the Fmoc (fluorenylmethyloxycarbonyl) process, the tBoc (t-butyloxycarbonyl) process, etc., or may be prepared using peptide synthesizers commercially available. It is preferred to prepare the protein or fusion protein by inserting the nucleic acid described above, especially the DNA incorporated into an expression vector, into a suitable expression system using an appropriate host cell selected from prokaryotes and eukaryotes, by genetic recombinant technology.

Examples of the host cell include microorganisms such as bacteria of the genus Escherichia, bacteria of the genus Corynebacterium, bacteria of the genus Brevibacterium, bacteria of the genus Bacillus, bacteria of the genus Serratia, bacteria of the genus Pseudomonas, bacteria of the genus Arthrobacter, bacteria of the genus Erwinia, bacteria of the genus Methylobacterium, bacteria of the genus Rhodobacter, microorganisms of the genus Streptomyces, microorganisms of the genus Zymomonas, yeasts of the genus Saccharomyces, etc.; animal cells including insect cells such as Bombyx mori, HEK293 cells, MEF cells, Vero cells, HeLa cells, CHO cells, W138 cells, BHK cells, COS-7 cells, MDCK cells, C127 cells, HKG cells, human kidney cell line; and the like.

The method of introducing the expression vector into the host cell can be performed in accordance with the methods described in the manuals of experimental operations including Maniatis et al. supra, for example, the electroporation method, the protoplast method, the alkaline metal method, the calcium phosphate precipitation method, the DEAE dextran method, the microinjection method, the particle gun method, etc. Use of inset cells such as Sf9, Sf21, etc. is described in Baculovirus Expression Vectors, A Laboratory Manual (W. H. Freeman and Company), New York, 1992), Bio/Technology, 1988, 6, 47; etc.

The protein of the present invention or the fusion protein containing the protein of the invention may be obtained by expressing the expression vector described above in the host cells above, and recovering and purifying the objective protein from the host cells or medium. To purify the protein, a suitable method is appropriately chosen from methods conventionally used for the purification of proteins. Specifically, a suitable method is appropriately chosen from methods conventionally used, such as salting-out, ultrafiltration, isoelectric point precipitation, gel filtration, electrophoresis; various affinity chromatographies including ion-exchange chromatography, hydrophobic chromatography, antibody chromatography, etc., chromato-focusing, adsorption chromatography, reversed-phase chromatography, and the like. Purification may then be performed in a suitable order, if necessary, using the HPLC system, etc.

Where the protein of the invention is expressed as the fusion protein with a histidine tag, a FLAG tag, etc., it is preferred to use purification suitable for the tag. The fusion protein may also be recovered through cleavage with an appropriate protease (thrombin, trypsin, etc.). It is one of the methods for genetic engineering production to obtain the protein by the cell-free synthesis using a recombinant DNA molecule.

As described above, the protein of the invention can be produced in its own form or in the form of the fusion protein with other protein but the production is not limited thereto. The protein of the invention may also be converted into various forms. The protein may be modified by a variety of means known to those skilled in the art including, for example, various chemical modifications for proteins, binding to high molecular weight substances such as polyethylene glycol, etc., binding to insoluble carriers, inclusion into liposomes, and the like.

An antibody capable of specifically binding to the protein of the present invention includes immunospecific antibodies such as a monoclonal antibody, a polyclonal antibody, a chimeric antibody, a single-stranded antibody, a humanized antibody, etc., preferably a monoclonal antibody. Such antibodies can be produced by conventional procedures which involve using the protein of the present invention as an antigen, immunizing a non-human animal and recovering the sera, or inducing a hybridoma cell producing a monoclonal antibody. These antibodies may also be labeled in a conventional manner, using a fluorescent substance, e.g., FITC (fluorescein isocyanate) or tetramethylrhodamine isocyanate, a radioactive isotope, an enzyme protein such as alkaline phosphatase, peroxidase, etc.

The protein of the present invention may be used as a marker protein in various molecular biological methods using GFP known heretofore or its mutants as a marker, in place of the GFP or mutants thereof. In various vectors commercially available as a vector capable of readily expressing the GFP-fused protein used by a person skilled in the art, for example, the pRSET/EmGFP vector manufactured by Invitrogen Corp and the like, when ORF encoding GFP is replaced with that of the present protein and the resulting vector is prepared, the fusion protein of an optional protein to the protein of the invention can be produced or utilized in a simple manner. By the use of such vectors, the in vivo or intracellular expression of the optional protein and the intracellular and/or extracellular localization of the optional protein can be determined or confirmed. The determination or confirmation can be made by detecting or measuring the fluorescence emission of the protein of the present invention or the fusion protein containing the protein of the invention.

Further by ligating the nucleic acid encoding the protein of the present invention or the fusion protein containing the protein of the invention under control of an optional functional nucleic acid and detecting or measuring the fluorescence emission from the protein of the present invention or the fusion protein containing the protein of the invention, the control mechanism of the functional nucleic acid can be examined or a substance for promoting or inhibiting the function of the functional nucleic acid can be surveyed.

### EXAMPLE 1

### Preparation of UMFP-1 (ECFP-W66F-S72A-S175G-A206K)

ECFP (mutant fluorescent protein comprising the amino acid sequence represented by SEQ ID NO: 1)-encoding DNA (ECFP gene) on pcDNA3 manufactured by Invitrogen Corp. was excised out with restriction enzymes BamHI and EcoRI and recombined into plasmid vector pRSETB (Invitrogen Corp.) linearized with the restriction enzymes to construct pRSETB/ECFP. Using this plasmid vector as a template, the 3 amino acid mutations of S72A, S 175G and A206K were introduced using the following primer DNAs by the method of Asano et al. (Nuc. Acid Res., 2000, 28 (16), e78).
Primer 1: CAGTGCTTCAGCCGCTACCCC (SEQ ID NO: 3)
Primer 2: GAGGACGGCAGCGTGCAGCTC (SEQ ID NO: 4)
Primer 3: ACCCAGTCCGCCCTGAGCAAA (SEQ ID NO: 5)

That is, 20 µL containing 500 ng of pRSETB/ECFP, 10 pmol each of Primers 1 to 3, 3.75 nmol of dNTPs, 1.25U of Pfu DNA polymerase and 20 U of Pfu DNA ligase (STRATAGENE Corp.) was prepared and pre-incubation was performed at 65°C for 5 minutes to repair the nick in the template DNA with Pfu DNA ligase. After DNA denaturation at 95°C for a minute, a total of 20 cycles were performed: one cycle included DNA denaturation at 95°C for 10 seconds, annealing at 55°C for 30 seconds and extension/ligation at 65°C for 10 minutes. After the thermal cycling reaction, 0.4 µL (8U) of DpnI (New England BioLabs, Inc.) was added to 20 µL of the reaction solution, followed by incubation at 37°C for an hour. After extraction with phenol-chloroform to purify the DNA, Escherichia coli JM109 (DE3) was transformed with the DNA by the calcium chloride method to give plasmid vector pRSETB/mSECFP bearing the DNA (SEQ ID NO: 14) encoding ECFP-S72A-S175G-A206K (SEQ ID NO: 15).

Using this vector as a template, a thermal cycling reaction was carried out using the following primer.
Primer 4: ACCCTGACCTTCGGCGTGCAG (SEQ ID NO: 6)

The conditions for the thermal cycling reaction were the same as those for the thermal cycling reaction shown above, except for the primer used. This reaction was performed to construct vector pRSETB/UMFP-1 encoding histidine-tagged ECFP-W66F-S72A-S175G-A206K (UMFP-1). The nucleotide sequence and amino acid sequence of UMFP-1 are represented by SEQ ID NO: 16 and SEQ ID NO: 17, respectively.

Using this vector, Escherichia coli JM109 (DE3) transformed by the calcium chloride method was cultured in 2 mL of LB liquid medium supplemented with 100 µg/mL of ampicillin at 23°C for 4 days. The cells obtained were lysed by French press. The cell debris after lysis was removed by centrifugal separation, and the supernatant was applied on a nickel chelate column (manufactured by Qiagen, Inc.). Histidine-tagged ECFP-W66F-S72A-S175G-A206K was recovered by eluting with 50 mM Tris hydrochloride buffer, pH 7.4, containing 100 mM imidazole and 300 mM NaCl. The buffer was further replaced with 50 mM HEPES buffer, pH 7.4, through a PD-10 desalting/buffer exchange column (GE Healthcare Bio-Sciences, Inc.) to give histidine-tagged ECFP-W66F-S72A-S175G-A206K (UMFP-1) (approximately 7.5 mg/mL).

### EXAMPLE 2

### Production of UMFP-2(ECFP-F46L-W66F-S72A-S175G-A206K-T65Q-Y145CT-H148S)

Using pRSETB/UMFP-1 prepared in EXAMPLE 1 as a template, a thermal cycling reaction was performed using the primer DNAs described below.
Primer 5: ACCACCCTGCAATTCGGCGTG (SEQ ID NO: 7)
Primer 6: GAGTACAACGGGATCAGCCAC (SEQ ID NO: 8)
Primer 7: GGGATCAGCTCAAACGTCTAT (SEQ ID NO: 9)

That is, 20 µL containing 500 ng of pRSETB/UMFP-1, 10 pmol each of Primers 5 to 7, 3.75 nmol of dNTPs, 1.25U of Pfu DNA polymerase and 20U of Pfu DNA ligase (STRATAGENE Corp.) was prepared, and pre-incubation was performed at 65°C for 5 minutes to repair the nick in the template DNA with Pfu DNA ligase. Then, after DNA denaturation at 95°C for a minute, a thermal cycling reaction was performed for 20 cycles, wherein one cycle included DNA denaturation at 95°C for 10 seconds, annealing at 55°C for 30 seconds and extension/ligation at 65°C for 10 minutes. After the thermal cycling reaction, 0.4 µL (8U) of DpnI (New England BioLabs, Inc.) was added to 20 µL of the reaction solution from the thermal cycling reaction, followed by incubation at 37°C for an hour. After further extraction with phenol-chloroform to purify the DNA, Escherichia coli JM109 (DE3) was transformed with the DNA by the calcium chloride method to give plasmid vector pRSETB/ECFP-W66F-S72A-S175G-A206K-T65Q-Y145G-H148S bearing the DNA (SEQ ID NO: 18) encoding ECFP-W66F-S72A-S175G-A206K-T65Q-Y145V-H148S (SEQ ID NO: 19).

Using this vector as a template, a thermal cyclic reaction was performed using the following primer to give pRSETB/UMFP-2.
Primer 8: ACCCTGAAGCTCATCTGCACC (SEQ ID NO: 10)

The conditions for the thermal cycling reaction were the same as those for the thermal cycling reaction shown above, except for the primer used. The same procedures as in EXAMPLE 1 were conducted using pRSETB/UMFP-2 to give histidine-tagged UMFP-2 (about 9.3 mg/mL). The nucleotide sequence and amino acid sequence of UMFP-2 are represented by SEQ ID NO: 20 and SEQ ID NO: 21, respectively.

### EXAMPLE 3

### Production of UMFP-3 (ECFP-F46L-W66F-S72A-S175G-A206K-T65Q-Y145G-H148S-T203V)

Using pRSETB/UMFP-2 produced in EXAMPLE 2 as a template, a thermal cycling reaction was carried out using the following primer DNA to give pRSETB/UMFP-3.
Primer 9: TACCTGAGCGTCCAGTCCGCC (SEQ ID NO: 11)

The conditions for the thermal cycling reaction were the same as those for the thermal cycling reaction shown above, except for the primer used. The same procedures as in EXAMPLE 1 were conducted using this pRSETB/UMFP-3 to give histidine-tagged UMFP-3 (about 9.3 mg/mL). The nucleotide sequence and amino acid sequence of UMFP-3 are represented by SEQ ID NO: 22 and SEQ ID NO: 23, respectively.

### EXAMPLE 4

### Measurement of the excitation peaks and emission peaks of UMFP

Aqueous solutions of UMFP-1 to 3/50 mM HEPES buffer, pH 7.4, prepared in EXAMPLES 1 to 3 were diluted to 100-fold, respectively, using 50 mM HEPES buffer (pH 7.4), and excitation spectra and fluorescence spectra were measured using a fluorospectrophotometer (HITACHI F-2500). At the same time, the excitation spectra and fluorescence spectra of wtGFP and known artificial GFP mutants, BFP (Heim et al., Non-Patent Literature 1 supra), CFP (Heim et al., Curr. Biol., 1996, 6, 178-182), YFP (Ormoe et al., 1994, Science, 273, 1392-1395) and DsRed (Terskikh et al., 2000, Science, 290, 1585-1588) were measured under the same conditions as in UMFP. The fluorescence spectra in which the maximum brightness of each fluorescent protein is normalized to 1 are illustrated in FIG. 2. All of UMFP-1 to 3 showed the excitation peak at approximately 355 nm and the emission peak at approximately 424 nm.

### EXAMPLE 5

### pH-Independent fluorescence intensities of UMFP-3

Using 50 mM glycine-HCl buffer (pH 3.0 to 3.4), 50 mM NaOAc (pH 3.8 to 5.4), 50 mM MES (pH 5.8 to 6.2), 50 mM MOPS (pH 6.6 to 7.0), 50 mM HEPES (pH 7.4 to 7.8) and 50 mM glycine (pH 8.6 to 9.0), buffers ranging from pH 3.0 to 9.0 were prepared. The fluorescence of 2 µM of UMFP-3 in 20 mM of each buffer was measured and the fluorescence intensity at each pH was calculated. The same measurement was conducted on GFP and BFP as well. The results are shown in FIG. 3.

In both GFP and BFP, the fluorescence intensities decreased to 1/2 or less in an acidic environment. On the other hand, the fluorescence intensities of UMFP-3 were constant over a wide range of pH (pH 3.0 to 9.0).

### EXAMPLE 6

### Photostability of UMFP-3

Photostability was measured by transient expression of UMFP-3 and EBFP for comparison on HeLa cells. Each of pcDNA3/UMFP-3 and pcDNA3/EBFP was transfected to HeLa cells cultured on a 35 mm glass-bottomed dish, using Surperfect (Invitrogen). One day after the transfection, each recombinant protein was confirmed to be normally expressed in the cytoplasm and the photostability was then assessed. A microscope used was a Nikon TE-2000E inverted microscope, equipped with a Fluor 40x objective lens and a 1.3 NA oil-immersion objective. The fluorescence from UMFP-3 and EBFP was excited in the range of wavelengths between 340 and 380 nm and detected with a band pass filter at wavelengths between 435 and 485 nm. FIG. 4 shows the attenuation curves of the fluorescence intensities obtained by repeating the procedure to expose HeLa cells bearing each of the fluorescent proteins UMFP-3 and EBFP expressed to the excitation light for 10 seconds and take pictures. As shown in FIG. 4, when the emission intensity after 1000 seconds to the emission intensity immediately after pulse irradiation for 10 seconds was measured, the fluorescent protein of the invention maintained approximately 80% of the emission intensity, whereas known EBFP maintained only approximately 10%. The figure also shows that the fluorescent protein of the present invention can provide linear attenuation at least within 1000 seconds after irradiation.

### EXAMPLE 7

### Production of the FRET pair using UMFP-3 as a donor and ECFP as an acceptor and examination of the efficiency of FRET

For measurement of the fluorescence spectra and FRET efficiency of UMFP-3 and ECFP, a chimeric protein was constructed by binding UMFP-3 deleted of 11 amino acids from the C terminus to mSECFP deleted of 4 amino acids from the N terminus through 2 linker amino acids of leucine and glutamic acid as the recognition sequences of restriction enzyme XhoI (hereinafter CΔ11UMFP-LE-NΔ4ECFP: the nucleotide sequence and amino acid sequence are shown bin SEQ ID NO: 24 and SEQ ID NO: 25, respectively). Using a sense primer containing the recognition sequence of restriction enzyme BamHI and an antisense primer containing the recognition sequence of restriction enzyme XhoI, PCR was performed to amplify cDNA of CΔIIUMFP. Likewise, cDNA of NΔ4ECFP was amplified by performing PCR using a sense primer containing the recognition sequence of restriction enzyme XhoI and an antisense primer containing the recognition sequence of restriction enzyme EcoRI. The restriction enzyme-treated product was introduced into pRSETB (Invitrogen) to construct Escherichia coli expression plasmid pRSETB/CΔ11UMFP-LE-NΔ4ECFP. pRSETB/CΔ11UMFP-LE-NA4ECFP, pRSETB/UMFP-3 and pRSETB/ECFP were transfected to Escherichia coli JM109 (DE3) to express the respective recombinant proteins at room temperature, followed by purification using polyhistidine tag. The fluorescence spectra of the samples purified were measured using an F-2500 fluorospectrophotometer (HITACHI). The measurement was conducted with a solution of each sample dissolved in 50 mM HEPES (pH 7.4) in a concentration of 2 µM. FIG. 5 shows the fluorescence spectra of CΔ11UMFP-LE-NΔ4ECFP (blue), UMFP-3 (red) and ECFP (cyan) when excited with excitation light at 355 nm. Based on this experiment, the FRET efficiency of CΔ11UMFP-LE-NΔ4ECFP is calculated to be approximately 66%. It is described that the cAMP indicator using the FRET pair of CFP-YFP, which is commonly used because of a good efficiency of FRET, has approximately a few % of the FRET efficiency in view of the fluorescence spectra (Literature: Ponsioen, B. et al. Detecting cAMP-induced Epac activation by fluorescence resonance energy transfer: Epac as a novel cAMP indicator. EMBO Rep. 5, 1176-1180 (2004)). Therefore, this fluorescent protein pair was capable of providing FRET in an extremely high efficiency by excitation light of lower wavelengths than those used heretofore.

### EXAMPLE 8

### Real-time imaging of caspase-3 activation by SCAT type indicators using the FRET pair of UMFP-3 and mSECFP

Using FRET from UMFP-3 to mSECFP, UC-SCAT3 (the nucleotide sequence and amino acid sequence are shown by SEQ ID NO: 26 and SEQ ID NO: 27, respectively), which is an indicator for the activation of caspase-3, which is a cysteine protease, was prepared. Plasmid UC-SCAT3-pcDNA3.1(-) expressing the indicator gene of the present invention was prepared by cleaving the Venus gene of SCAT3, which is an indicator for activation of caspase-3 using ECFP and Venus as the FRET pair (Takemoto K, Nagai T, Miyawaki A, et al.: Spatio-temporal activation of caspase revealed by indicator that is insensitive to environmental effects. J. Cell Biol. 160: 235-243, 2003) with restriction enzymes KpnI and HindIII, introducing UMFP-3 thereinto, further cleaving the ECFP gene with restriction enzymes BamHI and KpnI and introducing mSECFP thereinto. Using Surperfect (Invitrogen), UC-SCAT3-pcDNA3.1(-) was transfected to HeLa cells cultured on a 35 mm glass-bottomed dish to express on the cytoplasm. Imaging was performed one day after the transfection. To induce apoptosis, the cells were treated with 50 ng/ml of TNFα and 10 µg/ml of cycloheximide, 90 minutes before imaging. The cells were observed using a Nikon TE-2000E inverted microscope equipped with an Apo-VC 60x objective lens and a 1.35 NA oil-immersion objective lens. UC-SCAT3 expressed on the cytoplasm of HeLa cells was excited in the wavelength region of 352 to 388 nm. The fluorescence was detected using a band pass filter in the wavelength region of 415 to 455 nm for UMFP-3 and for ECFP using a band pass filter in the wavelength region of 459 to 499 nm. The left side of FIG. 6 indicates changes in the ratio of UMFP-3/mSECFP accompanied by the activation of caspase-3 within each circular frame in the right side (ROI: Region of Interst). The abscissa shows lapse of time from the start of observation. The figure reveals that the activation of caspase-3 can be detected with changes in the fluorescence intensity ratio of UMFP-3/mSECFP.

### EXAMPLE 9

### Production of UMFP-4 (ECFP-F46L-T65Q-W66F-Q69L-S72A-Y145G-H148S-S175G-A206K-T203V)

Using as a template the plasmid vector pRSETB/UMFP-3 encoding UMFP-3 (ECFP-F46L-T65Q-W66F-S72A-Y145G-H148S-S175G-A206K-T203V) produced in EXAMPLE 3, a thermal cycling reaction was carried out using the following primer DNA.
Primer 10: 5'-TTCGGGGTGCTGTGCTTCGCC-3' (SEQ ID NO: 12)

That is, 20 µL containing 50 ng of pRSETB/UMFP-3, 10 pmol of Primer 10, 3.75 nmol of dNTPs, 1.25U of Pfu DNA polymerase and 20U of Pfu DNA ligase (STRATAGENE Corp.) was prepared, and pre-incubation was performed at 65°C for 5 minutes to repair the nick in the template DNA with Pfu DNA ligase. Then, after DNA denaturation at 95°C for a minute, a thermal cycling reaction was performed for 20 cycles, wherein one cycle included DNA denaturation at 95°C for 10 seconds, annealing at 55°C for 30 seconds and extension/ligation at 65°C for 10 minutes. After the thermal cycling reaction, 0.4 µL (8U) of DpnI (New England BioLabs, Inc.) was added to 20 µL of the reaction solution from the thermal cycling reaction, followed by incubation at 37°C for an hour. After further extraction with phenol-chloroform to purify the DNA, Escherichia coli JM109 (DE3) was transformed with the DNA by the calcium chloride method to give plasmid vector pRSETB/UMFP-4 bearing the DNA encoding ECFP-F46L-T65Q-W66F-Q69L-S72A-Y145G-H148S-S175G-A206K-T203V (UMFP-4) The nucleotide sequence and amino acid sequence of UMFP-4 are shown by SEQ ID NO: 28 and SEQ ID NO: 29, respectively.

Using this vector pRSETB/UMFP-4, Escherichia coli JM109 (DE3) transformed by the calcium chloride method was cultured in 200 mL of LB liquid medium supplemented with 100 µg/mL of ampicillin at 23°C for 4 days. The cells obtained were lysed by French press. The cell debris after lysis was removed by centrifugal separation, and the supernatant was applied on a nickel chelate column (manufactured by Qiagen, Inc.). Histidine-tagged ECFP-F46L-T65Q-W66F-Q69L-S72A-Y145G-H148S-S175G-A206K-T203V was recovered by eluting with 50 mM Tris hydrochloride buffer, pH 7.4, containing 100 mM imidazole and 300 mM NaCl. The buffer was further replaced with 50 mM HEPES buffer, pH 7.4, through a PD-10 desalting/buffer exchange column (GE Healthcare Bio-Sciences, Inc.) to give approximately 500 µg/mL of histidine-tagged ECFP-F46L-T65Q-W66F-Q69L-S72A-Y145G-H148S-S175G-A206K-T203V (UMFP-4).

### EXAMPLE 10

### Production of Sirius (ECFP-F46L-T65Q-W66F-Q69L-S72A-Y145G-H148S-S175G-A206K-T203V-F223S)

Using as a template the plasmid vector pRSETB/UMFP-4 encoding ECFP-F46L-T65Q-W66F-Q69L-S72A-Y145G-H148S-S175G-A206K-T203V produced in EXAMPLE 9, a thermal cycling reaction was performed using the following primer DNA.
Primer 11: 5'-TTCGGGGTGCTGTGCTTCGCC-3' (SEQ ID NO: 13)

That is, 20 µL containing 50 ng of pRSETB/UMFP-4, 10 pmol each of Primer 11, 3.75 nmol of dNTPs, 1.25U of Pfu DNA polymerase and 20U of Pfu DNA ligase (STRATAGENE Corp.) was prepared, and pre-incubation was performed at 65°C for 5 minutes to repair the nick in the template DNA with Pfu DNA ligase. Then, after DNA denaturation at 95°C for a minute, a thermal cycling reaction was performed for 20 cycles, wherein one cycle included DNA denaturation at 95°C for 10 seconds, annealing at 55°C for 30 seconds and extension/ligation at 65°C for 10 minutes. After the thermal cycling reaction, 0.4 µL (8U) of DpnI (New England BioLabs, Inc.) was added to 20 µL of the reaction solution from the thermal cycling reaction, followed by incubation at 37°C for an hour. After further extraction with phenol-chloroform to purify the DNA, Escherichia coli JM109 (DE3) was transformed with the DNA by the calcium chloride method to give plasmid vector pRSETB/Sirius bearing the DNA encoding ECFP-F46L-T65Q-W66F-Q69L-S72A-Y145G-H148S-S175G-A206K-T203V-F223S (Sirius). The nucleotide sequence and amino acid sequence of Sirius are shown by SEQ ID NO: 30 and SEQ ID NO: 31, respectively.

Using this vector pRSETB/Sirius, Escherichia coli JM109 (DE3) transformed by the calcium chloride method was cultured in 200 mL of LB liquid medium supplemented with 100 µg/mL of ampicillin at 23°C for 4 days. The cells obtained were lysed by French press. The cell debris after lysis was removed by centrifugal separation, and the supernatant was applied on a nickel chelate column (manufactured by Qiagen, Inc.). Histidine-tagged ECFP-F46L-T65Q-W66F-Q69L-S72A-Y145G-H148S-S175G-A206K-T203V-F223S (Sirius) was recovered by eluting with 50 mM Tris hydrochloride buffer, pH 7.4, containing 100 mM imidazole and 300 mM NaCl. The buffer was further replaced with 50 mM HEPES buffer, pH 7.4, through a PD-10 desalting/buffer exchange column (GE Healthcare Bio-Sciences, Inc.) to give approximately 500 µg/mL of histidine-tagged ECFP-F46L-T65Q-W66F-Q69L-S72A-Y145G-H148S-S175G-A206K-T203V-F223S (Sirius).

### EXAMPLE 11

### Observation of phagocytosis of Sirius-expressing E. coli by Dictyostelium discoideum through a two-photon excitation microscope

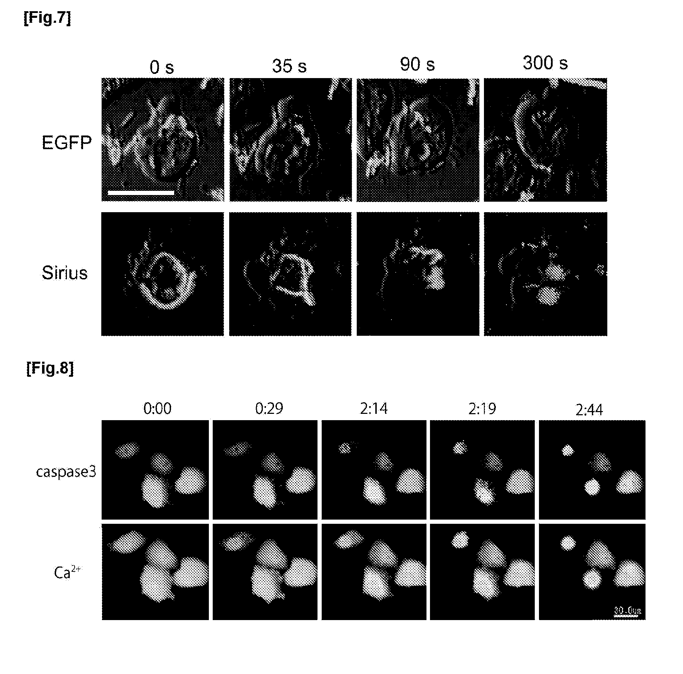

Dictyostelium discoideum AX2 was previously cultured in 10 mL af HL5 medium at 23.3°C. E. coli JM109 (DE3) was transformed by the calcium chloride method using the plasmid vector pRSETB/Sirius prepared in EXAMPLE 10 and then cultured at 37°C for 12 hours in 2 mL of LB medium supplemented with 100 µg/mL of ampicillin. Dictyostelium discoideum AX2 was precipitated by centrifugation and resuspended in BSS buffer. Sirius-expressing E. coli JM109 (DE3) was precipitated by centrifugation and resuspended in PBS buffer. The suspension of Dictyostelium discoideum AX2 in the buffer was mixed with the suspension of Sirius-expressing E. coli JM109 (DE3) in the buffer on 1% agarose gel on a 35 mm Petri dish. Thereafter, the mixture was incubated at room temperature for an hour, and the agarose gel on the Petri dish was inverted for microscopic observation of E. coli JM109 (DE3) expressing the mixed Dictyostelium discoideum AX2 and Sirius. A microscope used was a multiphoton excitation microscope Olympus Fluoview FV300, equipped with an UPlan FLN 40x objective lens and 1.30 NA 40x oil-immersion lens (Olympus). Using a Ti: sapphire laser (MAITAI, Spectra Physics, Inc.), Sirius was subjected to two-photon excitation by the excitation light at 780 nm for fluorescence observation. FIG. 7 shows the process where Sirius-expressing Escherichia coli is incorporated into Dictyostelium discoideum and digested via phagocytosis. This figure reveals that the state of E. coli taken up into phagosomes in Dictyostelium discoideum under acidic conditions can be clearly confirmed by using Sirius, having a pH-independent fluorescence intensity, as compared to EGFP having a pH-dependent fluorescence intensity.

### EXAMPLE 12

### Observation of dual FRET under single-wavelength excitation with quadruple wavelength emission spectrophotometry by concurrent use of the FRET pair using Sirius as a donor and mSECFP as an acceptor and the FRET pair using Sapphire as a donor and DsRed as an acceptor

First, using FRET from Sirius to mSECFP, SC-SCAT3 (the nucleotide sequence and amino acid sequence are shown by SEQ ID NO: 32 and SEQ ID NO: 33, respectively), which is an indicator for the activation of caspase-3, which is a cysteine protease, was prepared. Plasmid SC-SCAT3-pcDNA3.1(-) expressing the indicator gene of the present invention was prepared by cleaving the UMFP-3 gene of UC-SCAT3 prepared in EXAMPLE 8 with restriction enzymes KpnI and HindIII and introducing Sirius thereinto.

Two FRET pairs of SC-SCAT3 as an indicator of caspase-3 and SapRC2 as an indicator of calcium ions (Mizuno H, Sawano A, Miyawaki A, et al.: Red Fluorescent protein from Discosoma as a Fusion Tag and a Partner for Fluorescence Resonance Energy Transfer. Biochemistry, 40: 2502-2510, 2001) were expressed in HeLa cells. That is, using 4 µl of Surperfect (Invitrogen), 1 µg/dish of pcDNA3.1 (-)/SC-SCAT3 and pcDNA3/SapRC2 were introduced into HeLa cells cultured on a 35 mm glass-bottomed dish to effect co-expression.

Observation was made 2 days after the introduction of the plasmid vector. In order to induce apoptosis in HeLa cells, the cells were treated with 50 ng/ml of TNFα and 10 µg/ml of cycloheximide immediately before imaging. Wide-field fluorescence observation was conducted using a TE-2000E inverted microscope (Nikon) equipped with an Apo-VC 60x objective and a 1.35 NA 60x oil-immersion lens (Nikon). Interference filters used were all from Semrock, Inc.

For observation of fluorescence emission from SC-SCAT3 and SapRC2, excitation was performed using a mercury arc lamp as the light source, a FF01-370/36 as the excitation filter and CFW-Di0i-Clin as the dichroic mirror. The fluorescence emission of Sirius, mSEECFP, Sapphire and DsRed was detected through filters FF01-435/40, FF01/479/40, FF01-525/39 and FF01-585/40, respectively. FIG. 8 shows the images obtained when the activation of caspase-3 and Ca²⁺ kinetics in HeLa cells during apoptosis were observed with lapse of time. The figure reveals that using the caspase-3 indicator SC-SCAT3 and the calcium ion indicator SapRC2 co-expressed in the cells, the activation of caspase-3 and the concentration of calcium ions with lapse of time accompanied by induced cell death can be observed at the same time.

### INDUSTRIAL APPLICABILITY

First, the fluorescent protein of the invention emits fluorescence having an emission peak at 424 nm unknown heretofore and can be visually distinguished to the naked eye by its ultramarine color from other fluorescent proteins. Furthermore, the fluorescent protein of the invention has a pH-independent fluorescence intensity, which is not affected by pH changes, and these properties enable to use the fluorescent protein in an acidic environment proved to be difficult so far. Therefore, it makes possible to provide the fluorescent protein available under more diverse conditions in order to observe and confirm the localization of a specific substance visually in living organisms or cells by using the fluorescent protein of the present invention and can greatly contribute to studies of molecular biology.

## Claims

1. A fluorescent protein having an emission peak at 424 nm, comprising an amino acid sequence represented by SEQ ID NO: 1, in which each of the amino acid residues at the 66th position and the 175th position is substituted and at least one of the amino acid residues at the 72nd position and the 206th position is further substituted.

2. The fluorescent protein according to claim 1, wherein both of the amino acid residues at the 72nd position and the 206th position are substituted.

3. The fluorescent protein according to claim 2, wherein the amino acid residue at the 66th position is substituted with phenylalanine, the amino acid residue at the 72nd position with alanine, the amino acid residue at the 175th position with glycine and the amino acid residue at the 206th position with lysine, respectively.

4. The fluorescent protein according to any one of claims 1 to 3, wherein at least one of the amino acid residues at the 65th position, the 145th position and the 148th position is further substituted.

5. The fluorescent protein according to claim 4, wherein all of the amino acid residues at the 65th position, the 145th position and the 148th position are substituted.

6. The fluorescent protein according to claim 5, wherein the amino acid residue at the 65th position is substituted with glutamine, the amino acid residue at the 145th position with glycine and the amino acid residue at the 148th position with serine.

7. The fluorescent protein according to any one of claims 4 to 6, wherein the amino acid residue at the 46th position is further substituted.

8. The fluorescent protein according to claim 7, wherein the amino acid residue at the 46th position is substituted with leucine.

9. The fluorescent protein according to any one of claims 1 to 8, wherein the amino acid residue at the 203rd position is further substituted.

10. The fluorescent protein according to claim 9, wherein the amino acid residue at the 203rd position is substituted with valine.

11. The fluorescent protein according to claim 10, wherein the amino acid residue at the 66th position is substituted with phenylalanine, the amino acid residue at the 175th position with glycine, the amino acid residue at the 72nd position with alanine, the amino acid residue at the 206th position with lysine, the amino acid residue at the 65th position with glutamine, the amino acid residue at the 145th position with glycine, the amino acid residue at the 148th position with serine, the amino acid residue at the 46th position with leucine, and the amino acid residue at the 203rd position with valine.

12. The fluorescent protein having an emission peak at 424 nm according to claims 1 to 11, wherein one or more amino acids are deleted, substituted or added in the amino acid sequence.

13. A fused protein comprising the fluorescent protein according to any one of claims 1 to 12 and an optional protein or polypeptide.

14. A nucleic acid encoding the fluorescent protein or fused protein according to any one of claims 1 to 13.

15. A vector capable of expressing a fluorescent protein or fused protein encoded by the nucleic acid according to claim 14.

16. A host cell transformed or transfected with the expression vector according to claim 15.
